# EUROPÄISCHE PATENTSCHRIFT

(11) **EP 1 742 689 B1**
(45) Veröffentlichungstag und Bekanntmachung des Hinweises auf die Patenterteilung: **27.06.2007**
(21) Anmeldenummer: 05729351.6
(22) Anmeldetag: 19.04.2005
(51) Int. Cl.: A61M 5/315, A61M 5/24

(54) **VORRICHTUNG ZUR VERABREICHUNG EINES INJIZIERBAREN PRODUKTS MIT GESICHERTER DOSIEREINRICHTUNG**
DEVICE FOR ADMINISTERING AN INJECTABLE PRODUCT WITH A SECURED DOSING DEVICE
DISPOSITIF POUR ADMINISTRER UN PRODUIT INJECTABLE, A DISPOSITIF DE DOSAGE SECURISE

(30) Priorität: 23.04.2004 DE 102004020374
(43) Veröffentlichungstag der Anmeldung: 17.01.2007
(73) Patentinhaber: TecPharma Licensing AG, 3401 Burgdorf (CH)
(72) Erfinder: BURREN, Stefan, CH-3047 Bremgarten (CH); MOSER, Ulrich, CH-3412 Heimiswil (CH); SCHRUL, Christian, CH-3427 Utzenstorf (CH)
(86) Internationale Anmeldenummer: PCT/CH2005/000216
(87) Internationale Veröffentlichungsnummer: WO 2005/102420

(56) Entgegenhaltungen:
- EP-A- 0 496 141
- WO-A-97/36625
- DE-U1- 20 013 579
- US-A- 5 807 346
- US-B1- 6 235 004

## Beschreibung

Die Erfindung betrifft eine Vorrichtung zur Verabreichung eines injizierbaren Produkts, die eine freie Dosisauswahl eines Verwenders der Vorrichtung erlaubt. Die Vorrichtung ist insbesondere für solche Verwendungen geeignet, in denen der Verwender sich das Produkt selbst verabreicht und die Dosis pro Verabreichung selbst individuell auswählen, d. h. einstellen, kann. Besonders bevorzugt ist die Vorrichtung ein Injektionsgerät für beispielsweise die Verabreichung von Insulin in der Diabetestherapie oder die Verabreichung eines Wachstumshormons.

Aus der EP 0 713 403 A1 ist eine Spritze für die Verabreichung von flüssigen pharmazeutischen Mischungen und generell auch anderen Flüssigkeiten bekannt, die eine Einstellung einer pro Injektion zu verabreichenden Flüssigkeitsdosis einmalig erlaubt; genannt ist eine Einstellung durch einen Apotheker. Für den Patienten, der sich anschließend mit der Spritze die pharmazeutische Flüssigkeit verabreicht, ist es jedoch schwierig, die einmal eingestellte Dosis zu verändern. Hierdurch soll eine fehlerhafte Verwendung der Spritze verhindert werden. Eine derartige Spritze wird den Bedürfnissen nicht gerecht, da in vielen Therapien die optimale Produktdosis variiert, beispielsweise in Abhängigkeit von der Tageszeit, sportlichen Aktivitäten oder der Einnahme von Mahlzeiten.

Die Patentschrift US 5 807 346 A offenbart ein Injektionsgerät mit einer Dosiereinrichtung. Zur Dosierung wird ein Eingriffsglied in einen der Schlitze durch Rotation des Betätigungselements einrasten lassen. Während des gesamten Applikationsvorgangs dient der Schlitz schließlich als Führung des Eingriffsgliedes.

Die Patentschrift US 6 235 004 B1 offenbart ein Injektionsgerät mit einer Dosiereinrichtung. Zur Dosierung wird ein Betätigungselement im Uhrzeigersinn gedreht, wobei dieses sich dabei aus einem Gehäuse herausschraubt und dabei ein Einrastglied von Vertiefung zu Vertiefung springt bis die gewünschte Dosierung erreicht ist. Bei der anschließende Injektion wird das Betätigungselement in axiale Richtung in das Gehäuse gedrückt, wobei nun eine mögliche Rotation des Betätigungselements, hervorgerufen durch das Drehmoment einer sich nun im Uhrzeigersinn drehenden Kolbenstange, aufgrund der Verbindung des Einrastgliedes und der eingestellten Dosierung entsprechenden Vertiefung verhindert wird.

Vorrichtungen, die derartigen Anforderungen gerecht werden, sind beispielsweise aus der WO 97/36625 und DE 199 00 792 C2 bekannt. Die beiden Druckschriften betreffen Injektionsgeräte mit je einer Fördereinrichtung für die Ausschüttung des Produkts und einer Dosiereinrichtung für die Einstellung der Produktdosis, die bei einer nachfolgenden Injektion mittels der Fördereinrichtung förderbar und dadurch ausschüttbar ist. Die Fördereinrichtung umfasst einen Kolben, durch dessen Vorschub das Produkt aus einem Produktreservoir gefördert wird, eine Kolbenstange und ein Antriebsglied für die Kolbenstange. Das Antriebsglied und die Kolbenstange sind miteinander in solch einem Eingriff, dass eine Vorschubbewegung des Antriebsglieds eine ebensolche Vorschubbewegung der Kolbenstange bewirkt, das Antriebsglied jedoch eine Rückzugsbewegung in die Gegenrichtung bis in eine Auslöseposition ausführt, aus der heraus erneut eine Injektion vorgenommen werden kann. Die Auslöseposition wird mittels der Dosiereinrichtung bestimmt, die einen einstellbaren Dosieranschlag für das Antriebsglied bildet. Die bekannten Vorrichtungen haben sich in der Praxis bewährt, sollten die Gefahr von Fehldosierungen aber noch sicherer ausschließen.

Es ist eine Aufgabe der Erfindung, eine Vorrichtung zur Verabreichung eines injizierbaren Produkts zu schaffen, die eine freie Dosisauswahl bei verringerter Gefahr einer Fehldosierung erlaubt.

Die Erfindung betrifft eine Vorrichtung zur Verabreichung eines injizierbaren Produkts, vorzugsweise ein handliches, in einer Tasche der Kleidung tragbares Injektionsgerät. Die Vorrichtung umfasst ein Gehäuse mit einem Reservoir für das Produkt, eine Fördereinrichtung für die Förderung des Produkts und eine Dosiereinrichtung für die pro Injektion wahlfrei einstellbare Produktdosis. Das Reservoir kann unmittelbar von dem Gehäuse selbst gebildet werden. Vorzugsweise bildet das Gehäuse allerdings ein Aufnahmefach für ein Produktbehältnis, das vorzugsweise wie üblich als vorabgefüllte Ampulle in den Handel gelangt. Als "Gehäuse mit einem Reservoir" wird auch solch ein Gehäuse verstanden, das ein Aufnahmefach für ein Produktbehältnis bildet, in dem das Produktbehältnis noch nicht eingesetzt ist.

Die Fördereinrichtung wird von dem Gehäuse bewegbar gelagert. Sie kann eine Förderbewegung ausführen, durch die das Produkt aus dem Reservoir gefördert und ausgeschüttet wird. Sie kann relativ zu dem Gehäuse oder wenigstens einem Teil des Gehäuses in wenigstens, vorzugsweise genau zwei unterschiedliche Positionen bewegt werden, die vorgegeben sind, vorzugsweise durch Anschläge. Die eine der Positionen ist eine Auslöseposition, aus der heraus die Förderbewegung unmittelbar oder nach vorheriger Ausführung einer anderen Bewegung ausgeführt wird. Die andere der Positionen ist eine Freigabeposition, aus der die Fördereinrichtung in die Auslöseposition bewegbar ist. Die Förderbewegung und die Bewegung in die Auslöseposition sind bevorzugterweise Linearbewegungen, besonders bevorzugt entlang einer einzigen Translationsachse. Die Bewegung aus der Freigabeposition in die Auslöseposition ist der Förderbewegung vorzugsweise exakt entgegengerichtet. Vorzugsweise ist die Fördereinrichtung zwischen der Auslöseposition und der Freigabeposition hin und her bewegbar, besonders bevorzugt ist sie ausschließlich in dieser Art bewegbar. Die Bewegung aus der Auslöseposition wird vorzugsweise durch manuellen Druck auf die Fördereinrichtung und die Bewegung in die Auslöseposition wird vorzugsweise durch eine manuell aufgebrachte Zugkraft bewirkt. Die Bewegung in die Auslöseposition wird im Folgenden vereinfachend als Rücksetzbewegung bezeichnet. Wenn es heißt, dass die Fördereinrichtung eine Bewegung ausführt, so bedeutet dies nicht, dass im Falle einer bevorzugt mehrteiligen Ausführung der Fördereinrichtung sämtliche Teile der Fördereinrichtung die betreffende Bewegung stets oder überhaupt gemeinsam ausführen, obgleich eine gemeinsame Bewegung, zumindest phasenweise, bevorzugt wird.

Die Dosiereinrichtung ist so gelagert, vorzugsweise so mit dem Gehäuse verbunden, dass sie-relativ zu der Fördereinrichtung oder zumindest einem Teil der Fördereinrichtung eine Dosierbewegung ausführen kann, um die durch die Förderbewegung förderbare Produktdosis einzustellen. Die einstellbare Produktdosis wird durch Dosierpositionen vorgegeben, in die die Dosiereinrichtung bei der Dosierbewegung vorzugsweise verrastet. Der entsprechende Rasteingriff kann mit dem Gehäuse oder/und mit der Fördereinrichtung gebildet sein. Die Dosierung kann vorgenommen werden, wenn die Fördereinrichtung die Freigabeposition einnimmt, vorzugsweise nur dann. Bei den vorgegebenen Dosierpositionen kann es sich um nur zwei unterschiedliche Dosierpositionen handeln, um beispielsweise zu unterschiedlichen Tageszeiten zwei unterschiedliche Produktdosen zu verabreichen. In anderen Ausführungen sind mehr als zwei, vorzugsweise ist eine Vielzahl unterschiedlicher Dosierpositionen vorgesehen, um in Anpassung an unterschiedliche Situationen und/oder einer bezüglich der zu verabreichenden Produktdosen heterogenen Personengruppe die Möglichkeit individueller Dosiseinstellung zu gewähren.

Nach der Erfindung ist die Dosiereinrichtung mit der Fördereinrichtung mittels Blockiereingriff gekoppelt, wenn die Fördereinrichtung die Auslöseposition einnimmt und ferner auch während der Rücksetzbewegung der Fördereinrichtung. Im Blockiereingriff ist die Dosiereinrichtung relativ zu der Fördereinrichtung in der zuvor eingestellten Dosierposition gegen Dosierbewegungen blockiert. Eine erzwungene Bewegung aus der blockierten Dosierposition ist allenfalls durch eine außergewöhnliche Kraftanstrengung möglich und hat vorzugsweise eine derart weitgehende Beschädigung der Vorrichtung zur Folge, dass eine nachfolgende Produktverabreichung nicht mehr möglich ist. Vorteil der Erfindung ist, dass die Dosis in der Freigabeposition eingestellt wird, eine "Nachdosierung" in der Auslöseposition verhindert wird und auch die Rücksetzbewegung stabilisiert ist. Vorteilhaft ist insbesondere die Blockierung in der Auslöseposition, da die Vorrichtung dadurch unmittelbar im Zusammenhang mit der Verabreichung sicherer gehandhabt werden kann, weil die für die Verabreichung erforderlichen Handgriffe nicht versehentlich zu einer Dosierbewegung führen können. Obgleich bevorzugt wird, dass die Dosiereinrichtung im Blockiereingriff eng geführt wird, um Bewegungen quer zur Richtung der Rücksetzbewegung zu verhindern, kann grundsätzlich ein gewisses Spiel vorhanden sein, solange hierdurch die eingestellte Dosis nicht verstellt werden kann. Falls die Bewegung der Fördereinrichtung aus der Auslöseposition in die Freigabeposition der Rücksetzbewegung entgegengerichtet ist, wie dies bevorzugt wird, bringt die Erfindung, falls keine Gegenmaßnahmen getroffen werden, den weiteren Vorteil, dass auch diese Bewegung und somit die Förderbewegung gegen eine versehentliche Verstellung der eingestellten Dosis stabilisiert ist.

Die Auslöseposition wird, wie bereits erwähnt, vorzugsweise als Anschlagposition bestimmt. Zwecks Dosierung ist diese Anschlagposition in und gegen die Richtung der Förderbewegung und dadurch die maximale Weglänge der Förderbewegung verstellbar.

Die Fördereinrichtung und die Dosiereinrichtung bilden je einen Dosieranschlag, und die beiden Dosieranschläge begrenzen die Rücksetzbewegung der Fördereinrichtung und bestimmen dadurch die Auslöseposition. Dies bedeutet, dass eines aus Fördereinrichtung und Dosiereinrichtung, vorzugsweise die Dosiereinrichtung, einen bezüglich seiner Position veränderbaren Anschlag bildet. Bevorzugte Beispiele für die Bildung des verstellbaren Dosieranschlags werden in der WO 97/36625 und der DE 199 00 792 C1 offenbart. In kinematischer Umkehr könnten die in diesen Druckschriften offenbarten Dosieranschläge ihrem Verlaufe nach, d. h. als diskrete Dosieranschläge oder als kontinuierlich verlaufender Dosieranschlag, auch an der Fördereinrichtung gebildet sein, so dass ein Anschlagnocken an der Dosiereinrichtung genügt, der durch die Dosierbewegung in eine Dosierposition eingestellt wird.

In bevorzugten Ausführungen wird die Blockierung der Dosiereinrichtung durch den Eingriff wenigstens einer Führung und wenigstens eines Eingriffsglieds bewirkt. Sichergestellt wird, dass für jede der Dosierpositionen eines aus Fördereinrichtung und Dosiereinrichtung eine Führung und das andere ein Eingriffsglied bildet, die miteinander in dem Blockiereingriff sind. Wegen der mehreren unterschiedlichen Dosierpositionen sind entsprechend mehrere Führungen und/oder mehrere Eingriffsglieder vorgesehen, von denen wenigstens ein Paar in jeder der Dosierpositionen in dem Blockiereingriff ist. Die Führung oder das Eingriffsglied kann an der Fördereinrichtung starr gebildet, insbesondere einstückig, oder beweglich an der Fördereinrichtung angeordnet sein. Das von der Dosiereinrichtung gebildete Gegenglied kann an der Dosiereinrichtung starr geformt, insbesondere einstückig, oder beweglich an der Dosiereinrichtung angeordnet sein. Bei beweglicher Anordnung vollführen die Führung und das Eingriffsglied relativ zueinander die Rücksetzbewegung und zusätzlich eine Bewegung quer zu der Rücksetzbewegung, wenn sie sich in den Blockiereingriff bewegen. Entsprechendes gilt, wenn sie sich aus dem Blockiereingriff bewegen.

Der Eingriff zwischen der Führung und dem Eingriffsglied kann in der Freigabeposition der Fördereinrichtung gelöst oder als lösbarer Rasteingriff gebildet sein, der vorteilhafterweise gleichzeitig den lösbaren Rasteingriff für die Dosisauswahl in der Freigabeposition der Fördereinrichtung bilden kann. In der zuletzt genannten Variante schwächt sich der Blockiereingriff bei der Bewegung in die Freigabeposition bis auf den Rasteingriff ab; umgekehrt verstärkt sich der in der Freigabeposition bestehende Rasteingriff zu dem Blockiereingriff bei der Rücksetzbewegung der Fördereinrichtung.

Die Führung oder die bevorzugt mehreren Führungen kann oder können insbesondere je als Führungsnut oder vorragender Führungssteg gebildet sein, die oder der sich über nahezu die gesamte Weglänge der Rücksetzbewegung der Fördereinrichtung erstreckt. Die Führung oder mehreren Führungen sollte oder sollten sich im Falle eines starren Eingriffsglieds so nahe als möglich bis zu dem Eingriffsglied erstrecken, wenn die Fördereinrichtung die Freigabeposition einnimmt. Falls das Eingriffsglied oder die mehreren Eingriffsglieder in der Freigabeposition mit der Führung oder den mehreren Führungen in einem lösbaren Rasteingriff sind, erstreckt sich die Führung oder erstrecken sich die mehreren Führungen entsprechend über eine verlängerte Strecke.

Die Dosierbewegung umfasst vorzugsweise eine Drehbewegung der Dosiereinrichtung relativ zu der Fördereinrichtung um eine Drehachse. Die Dosierbewegung kann eine reine Drehbewegung sein. Sie kann auch eine überlagerte Dosierbewegung aus Drehbewegung und Translationsbewegung, in diesem Fall vorzugsweise entlang der Drehachse sein. Die Förderbewegung der Fördereinrichtung umfasst vorzugsweise eine Bewegung der Fördereinrichtung relativ zu der Dosiereinrichtung entlang der Drehachse. Die Förderbewegung kann insbesondere eine reine Linearbewegung entlang der Drehachse sein. Insbesondere in solchen Ausbildungen bietet es sich an, dass eine der Strukturen, nämlich die Fördereinrichtung oder die Dosiereinrichtung, das andere um die Drehachse wenigstens teilweise umgibt und die in der erforderlichen Anzahl vorhandenen Führungen und/oder Eingriffsglieder an einander zugewandt gegenüberliegenden Mantelflächen der Fördereinrichtung und der Dosiereinrichtung angeordnet sind. Diejenige der beiden Strukturen, vorzugsweise die Dosiereinrichtung, die die andere wenigstens teilweise umgibt, ist oder umfasst vorzugsweise einen Hülsenkörper und bildet vorzugsweise die wenigstens eine Führung oder die mehreren Führungen an ihrer Innenmantelfläche.

Die Fördereinrichtung kann einteilig sein, ist vorzugsweise jedoch mehrteilig. Sie umfasst in der mehrteiligen Ausbildung ein Förderglied, das die Förderbewegung ausführt und dabei unmittelbar auf das in dem Reservoir befindliche Produkt wirkt, und eine Antriebseinrichtung, die mit dem Förderglied gekoppelt ist, um dessen Förderbewegung zu bewirken. Die Antriebseinrichtung umfasst vorzugsweise ein Abtriebsglied und ein Antriebsglied, die relativ zueinander bewegbar und miteinander so gekoppelt sind, dass eine Antriebsbewegung des Antriebsglieds eine Abtriebsbewegung des Abtriebsglieds bewirkt. Das Abtriebsglied kann mit dem Förderglied steif verbunden sein oder ist mit dem Förderglied so gekoppelt, dass die Abtriebsbewegung des Abtriebsglieds die Förderbewegung bewirkt. Vorzugsweise nimmt das Abtriebsglied bei seiner Abtriebsbewegung das Förderglied einfach mit. Das Antriebsglied ist so gelagert, dass es zum Einen die Antriebsbewegung und zum Anderen eine der Antriebsbewegung entgegengerichtete Bewegung in die Auslöseposition der Fördereinrichtung ausführen kann. Das Antriebsglied und das Abtriebsglied sind vorzugsweise so miteinander gekoppelt, dass das Antriebsglied bei der Antriebsbewegung das Abtriebsglied mitnimmt, während das Antriebsglied die Bewegung in die Gegenrichtung vorzugsweise ohne das Abtriebsglied ausführt. Derartige Antriebseinrichtungen sind insbesondere von Zahnstangenpens bekannt, beispielsweise aus der WO 97/36625 und der DE 199 00 792 C2. Geeignet ist beispielsweise auch eine Antriebseinrichtung, wie sie die DE 199 45 397 C2 beschreibt, bei der das Abtriebsglied glatt und das Antriebsglied mit in die glatte Außenfläche des Abtriebsglieds sich stemmenden Eingriffselementen gebildet sind. Die Bewegungen der Fördereinrichtung und in der mehrteiligen Ausbildung der Glieder der Fördereinrichtung umfassen oder sind vorzugsweise Linearbewegungen entlang einer Translationsachse der Fördereinrichtung.

Falls die Dosiereinrichtung, wie bevorzugt, die wenigstens eine Führung bildet, ist es vorteilhaft, wenn ihr Dosieranschlag nicht an dem Ende der wenigstens einen Führung, sondern an einer anderen Stelle der Dosiereinrichtung gebildet ist. Falls die Fördereinrichtung, wie bevorzugt, das wenigstens eine Eingriffsglied umfasst, wird es bevorzugt, wenn das wenigstens eine Eingriffsglied nicht den Dosieranschlag der Fördereinrichtung bildet, sondern zusätzlich dazu vorgesehen ist. Andererseits kann das wenigstens eine Eingriffsglied durchaus den Dosieranschlag bilden, insbesondere dann, wenn an dem Ende der wenigstens einen Führung der für die Dosierung erforderliche Gegenanschlag, ebenfalls als Dosieranschlag bezeichnet, gebildet ist.

Die Fördereinrichtung ist vorzugsweise für eine manuelle Betätigung ausgebildet. Sie kann aber auch einen motorischen Antrieb umfassen, der die Förderbewegung bewirkt und in der Auslöseposition der Fördereinrichtung ausgelöst wird. In beiden Ausbildungen umfasst sie ein Betätigungselement, im einen Fall für die manuelle Betätigung und Bewirkung der Förderbewegung und im anderen Fall für das Auslösen des motorischen Antriebs. Für die manuelle Betätigung, wie sie für Injektionsgeräte bevorzugt wird, bringt der Verwender mittels des Betätigungselements die für die Förderbewegung erforderliche Förderkraft auf.

Vorteilhafterweise ist eine Weglänge, die das Betätigungselement bei der Betätigung ausführt, größer als eine Weglänge der Förderbewegung, die die Fördereinrichtung für eine vollständige Ausschüttung der eingestellten Produktdosis zurücklegt. Die Weglängenvergrößerung ist insbesondere dann von Vorteil, wenn die der eingestellten Produktdosis entsprechende Weglänge der Förderbewegung sehr kurz ist, beispielsweise einen oder wenige Millimeter oder gar weniger als einen Millimeter beträgt. Würde sich die Bewegung des Betätigungselements über eine ebenso kurze Weglänge nur erstrecken, wären Fehlinterpretationen dergestalt möglich, dass der Verwender glaubt, er habe die Produktdosis noch gar nicht oder nur unvollständig verabreicht.

Der gegenüber der Förderbewegung verlängerte Betätigungsweg des Betätigungselements kann getriebetechnisch so realisiert sein, dass die Bewegung des Betätigungselements mittels eines Untersetzungsgetriebes stets und kontinuierlich in die Förderbewegung untersetzt wird. Nicht zuletzt aus Gründen der Einfachheit umfasst der Betätigungsweg des Betätigungselements jedoch eine Leerbewegung des Betätigungselements ohne Förderbewegung und eine Bewegung des Betätigungselements gemeinsam, 1:1, mit der Förderbewegung.

In bevorzugten Ausführungen bildet das Betätigungselement entweder die Führung, die vorzugsweise aus mehreren Einzelführungen besteht, oder das Eingriffsglied, das ebenfalls von mehreren Eingriffsgliedern gebildet sein kann. Das Betätigungselement und die von ihm gebildete Führung oder das von ihm gebildete Eingriffsglied können aus einem Stück gebildet sein. Alternativ ist die Führung oder das Eingriffsglied mit dem Betätigungselement gelenkig verbunden, d. h. relativ zu dem Betätigungselement beweglich. Die Gelenkverbindung ist so gestaltet, dass sie eine Bewegung des Betätigungselements in eine Bewegung der Führung oder des Eingriffsglieds in und vorzugsweise auch wieder aus dem Blockiereingriff umwandelt. Die betreffende Bewegung des Betätigungselements ist vorzugsweise eine Bewegung, die relativ zu einem auf das Produkt wirkenden Förderglied der Fördereinrichtung stattfindet. Besonders vorteilhaft ist es, wenn die genannte Leerbewegung des Betätigungselements die Herstellung und vorzugsweise auch das Lösen des Blockiereingriffs bewirkt.

Insbesondere kann ein Kurvengelenk mit vorzugsweise genau zwei im Eingriff befindlichen Gelenkelementen die Gelenkverbindung bilden. Die wenigstens, vorzugsweise genau zwei Gelenkelemente des Kurvengelenks gleiten bei der Umwandlung der Bewegung des Betätigungselements in die Bewegung der Führung oder des Eingriffsglieds vorzugsweise aneinander ab, grundsätzlich kann die Bewegung im Gelenk jedoch eine Roll- oder Gleit-Roll-Bewegung sein.

Das Betätigungselement kann über das Eingriffsglied mit einem die Förderbewegung ausführenden Förderglied der Fördereinrichtung verbunden sein. Ist das Eingriffsglied mit dem Betätigungselement gelenkig verbunden, so ist es vorzugsweise auch mit dem Förderglied oder vorzugsweise einem anderen Glied der Fördereinrichtung, das die Bewegung des Betätigungselements auf das Förderglied überträgt, gelenkig verbunden. Für die in diesem Fall zweite Gelenkverbindung wird ein Drehgelenk bevorzugt. Alternativ oder zusätzlich zu einer zweiten Gelenkverbindung kann die Beweglichkeit des Eingriffsglieds durch material- und/oder formelastische Ausbildung des Eingriffsglieds erhalten werden.

Vorteilhafte Ausgestaltungen werden auch in den Unteransprüchen und deren Kombinationen beschrieben.

Bevorzugte Ausführungsbeispiele der Erfindung werden nachfolgend anhand von Figuren beschrieben. An den Ausführungsbeispielen offenbar werdende Merkmale bilden je einzeln und in jeder Merkmalskombination die Gegenstände der Ansprüche und auch die vorstehend beschriebenen Ausgestaltungen vorteilhaft weiter. Es zeigen:
- Figur 1: ein Injektionsgerät aus dem Stand der Technik,
- Figur 2: ein erfindungsgemäßes Antriebs- und Dosiermodul für das Injektionsgerät der Figur 1,
- Figur 3: das Antriebs- und Dosiermodul in einem Freigabezustand für eine Dosisauswahl,
- Figur 4: das Antriebs- und Dosiermodul mit blockierter Dosisauswahl,
- Figur 5: das Antriebs- und Dosiermodul in einem auslösebereiten Zustand,
- Figur 6: das Antriebs- und Dosiermodul nach einer Ausschüttung der ausgewählten Dosis,
- Figur 7: das Antriebs- und Dosiermodul in dem Freigabezustand für eine nächste Dosisauswahl,
- Figur 8: ein Detail des Moduls im Zustand der Figur 3,
- Figur 9: ein Detail des Moduls im Zustand der Figur 4,
- Figur 10: ein Detail des Moduls im Zustand der Figur 5,
- Figur 11: einen proximalen Abschnitt eines modifizierten Antriebs- und Dosiermoduls in dem Freigabezustand für die Dosisauswahl,
- Figur 12: das modifizierte Antriebs- und Dosiermodul in dem Freigabezustand und
- Figur 13: das modifizierte Antriebs- und Dosiermodul während des Übergangs in den auslösebereiten Zustand.

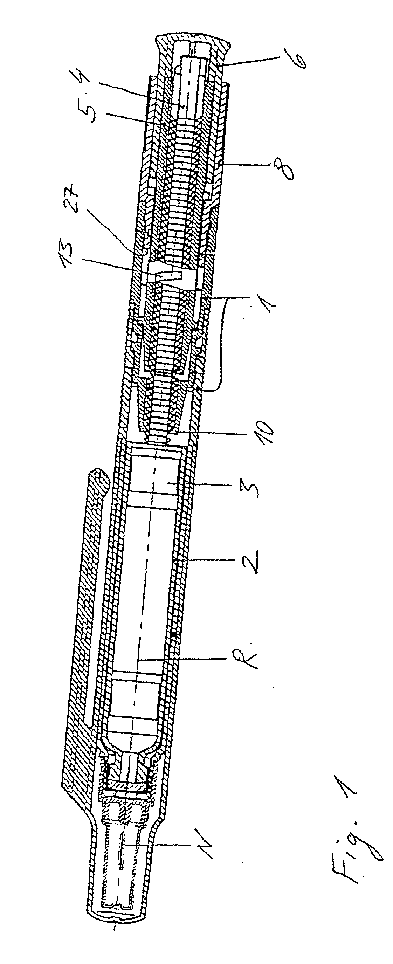

Figur 1 zeigt ein Injektionsgerät, das als Zahnstangenpen gebildet ist. Das Injektionsgerät umfasst ein zweiteiliges Gehäuse 1 aus einem distalen Gehäuseabschnitt und einem proximalen Gehäuseabschnitt, die miteinander fest verbunden sind, beispielsweise verschraubt. In einem Aufnahmefach des Gehäuses 1, das dessen distaler Gehäuseabschnitt bildet, ist ein Reservoir 2 aufgenommen. An einem distalen Auslass des Reservoirs 2 ist eine Injektionsnadel N befestigt. Die Längsachse der Injektionsnadel N bildet eine zentrale Längsachse R des Injektionsgeräts. Ein Kolben 3 verschließt das proximale Ende des Reservoirs 2. Der Kolben 3 kann entlang der Achse R auf den Auslass des Reservoirs 2 zu eine Förderbewegung ausführen, um Produkt aus dem Reservoir 2 zu verdrängen. Das Reservoir 2 ist eine handelsübliche Ampulle, die mit dem zu verabreichenden Produkt, beispielsweise Insulin, gefüllt ist.

Der Kolben 3 ist das unmittelbar auf das Produkt wirkende Förderglied einer Fördereinrichtung, die über den Kolben 3 hinaus ein Abtriebsglied 4, ein Antriebsglied 5 und ein Betätigungselement 6 umfasst. Wegen der Ausbildung des Förderglieds als Kolben 3 ist das unmittelbar auf den Kolben 3 wirkende Abtriebsglied 4 eine Kolbenstange und wird deshalb im Folgenden auch so bezeichnet. Die Kolbenstange 4 führt bei Betätigung der Fördereinrichtung 3-6 ebenfalls die Förderbewegung aus und drückt dabei den Kolben 3 in die distale Richtung. Die Kolbenstange 4 ist als Zahnstange gebildet mit mehreren in Richtung der Achse R sich erstreckenden Zahnreihen, die zur Verfeinerung der Dosisauswahl entlang der Achse R je um weniger als eine Zahnteilung zueinander versetzt sind. Das Antriebsglied 5 ist entlang der Achse R in die distale und die proximale Richtung bewegbar. Das Antriebsglied 5 und die Kolbenstange 4 sind so miteinander gekoppelt, dass das Antriebsglied 5 bei seiner Bewegung in die distale Richtung die Kolbenstange 4 mitnimmt, die Bewegung in die proximale Richtung jedoch ohne die Kolbenstange ausführt. Die Kopplung wird im Ausführungsbeispiel durch Eingriff von Mitnehmern in die Zahnreihen der Kolbenstange 4 bewirkt. Der Eingriff ist so, dass eine Bewegung der Kolbenstange 4 in die distale Richtung relativ zu dem Antriebsglied 5 verhindert und eine Bewegung des Antriebsglieds 5 in die proximale Richtung relativ zu der Kolbenstange 4 zugelassen wird. Um eine Mitnahme der Kolbenstange 4 bei der Bewegung in die proximale Richtung zu verhindern, bildet der proximale Abschnitt des Gehäuses 1 eine Rückhalteeinrichtung 10, die wie die Mitnehmer des Antriebsglieds 5 in wenigstens eine, im Ausführungsbeispiel in zwei Zahnreihen der Kolbenstange 4 eingreift, so dass die Kolbenstange 4 relativ zu dem Gehäuse 1 zwar in die distale Richtung, aber nicht in die proximale Richtung bewegt werden kann. Erreicht wird dies durch die Formung der Zähne der Zahnreihen als Sägezähne. Der proximale Abschnitt des Gehäuses 1 lagert die Kolbenstange 4 und das Antriebsglied 5 ferner so, dass diese Bestandteile der Fördereinrichtung 3-6 relativ zu dem Gehäuse 1 keine Drehbewegungen um die Achse R ausführen können.

Das Injektionsgerät erlaubt pro Injektion eine freie Auswahl der verabreichbaren Produktdosis. Für die Auswahl bzw. Einstellung der Produktdosis ist eine Dosiereinrichtung 8 vorgesehen, die relativ zu der Fördereinrichtung 3-6, insbesondere relativ zu deren Antriebsglied 5, eine Dosierbewegung ausführen kann. Der proximale Abschnitt des Gehäuses 1 lagert auch die Dosiereinrichtung 8 in einer für die Ausführung der Dosierbewegung geeigneten Weise. Im Ausführungsbeispiel, in dem die Dosierbewegung eine Drehbewegung um die Achse R ist, lagert der hintere Abschnitt des Gehäuses 1 die Dosiereinrichtung 8 um die Achse R drehbar. Die Achse R bildet somit die Translationsachse für die Fördereinrichtung 3-6 und die Rotationsachse für die Dosiereinrichtung 8. Die Dosiereinrichtung 8 ist bei Ausführung der Dosierbewegung zwischen diskreten, als Rastpositionen vorgegebenen Dosierpositionen bewegbar. Hierfür ist sie mit dem proximalen Abschnitt des Gehäuses 1 in jeder der Dosierpositionen in einem lösbaren Rasteingriff. Zu der Dosiereinrichtung 8 sei noch angemerkt, dass sie im Ausführungsbeispiel als Hülsenkörper gebildet ist und das Antriebsglied 5 sowie das Betätigungselement 6 umgibt. Das Antriebsglied 5 und das Betätigungselement 6 sind ebenfalls je als Hülsenkörper gebildet, wobei das Betätigungselement 6 einen proximalen Endabschnitt des Antriebsglieds 5 umgibt und für eine manuelle Betätigung der Fördereinrichtung 3-6 in die proximale Richtung aus der Dosiereinrichtung 8 hinausragt. Das Antriebsglied 5 schließlich umgibt die Kolbenstange 4.

Für die Einstellung der Produktdosis bilden das Antriebsglied 5 einen Dosieranschlag 13 und die Dosiereinrichtung 8 einen Dosieranschlag 27, der dem Dosieranschlag 13 in proximaler Richtung gegenüberliegt. Die Dosiereinrichtung 8 bildet ihren Dosieranschlag 27 mit einer distalen Stirnfläche, die wie in der DE 199 00 792 C2 beschrieben spiralig um die Achse R umläuft. Das Antriebsglied 5 bildet seinen Dosieranschlag 13 als radial nach außen abragenden Nocken, dessen Form dem Verlauf des spiraligen Dosieranschlags 27 angepasst ist.

In dem in Figur 1 dargestellten Zustand nimmt die Fördereinrichtung 3-6 im Gehäuse 1 eine distalste Position ein. In diesem Zustand wird die Produktdosis mittels der Dosiereinrichtung 8 eingestellt, indem ein der gewünschten Produktdosis entsprechender Abschnitt des Dosieranschlags 27 in die entlang der Achse R dem Dosieranschlag 13 gegenüberliegende Dosierposition bewegt wird. Der in der betreffenden Dosierposition zwischen den Dosieranschlägen 13 und 27 verbleibende, entlang der Achse R gemessene Abstand entspricht der Weglänge, d. h. dem Förderhub, die das Antriebsglied 5 gemeinsam mit der Kolbenstange 4 und dem Kolben 3 bei der Injektion zurücklegen kann. Nach der Einstellung der Produktdosis wird durch Ziehen an dem Betätigungselement 6 das Antriebsglied 5 und wegen des Eingriffs damit gemeinsam die Kolbenstange 4 in die proximale Richtung gezogen, bis der Dosieranschlag 13 in Kontakt mit dem Dosieranschlag 27 gelangt. Die Fördereinrichtung 3-6 nimmt dann eine Auslöseposition ein, aus der heraus sie für die Injektion durch Ausübung einer in die distale Richtung auf das Betätigungselement 6 wirkenden Druckkraft betätigt werden kann. Es ist klar, dass vor der Injektion die in Figur 1 dargestellte Gehäusekappe und ferner die Nadelschutzkappe entfernt werden müssen.

Der proximale Abschnitt des Gehäuses 1, die von diesem Abschnitt gelagerten Teile der Fördereinrichtung 3-6 und die mit dem Gehäuseabschnitt von der Dosierbewegung abgesehen fest verbundene Dosiereinrichtung 8 bilden ein Antriebs- und Dosiermodul, wie es aus der DE 199 00 792 C2 bekannt ist. Dieses Modul kann durch ein erfindungsgemäßes Antriebs- und Dosiermodul ersetzt werden.

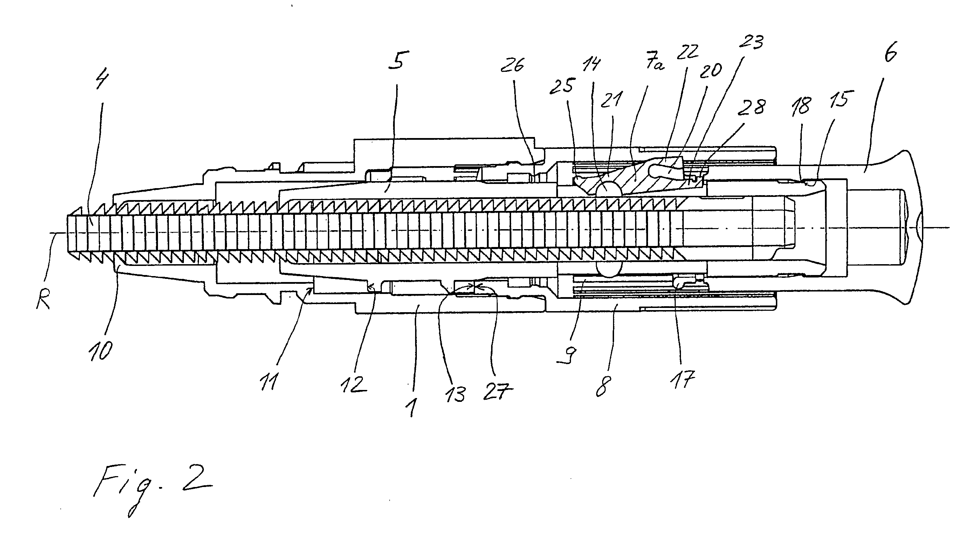

Figur 2 zeigt ein erfindungsgemäßes Antriebs- und Dosiermodul in einem ersten Ausführungsbeispiel. Teile gleicher Funktion wie die des Antriebs- und Dosiermoduls des bekannten Injektionsgeräts sind mit den gleichen Bezugszeichen versehen. Soweit Ausführungen zum erfindungsgemäßen Antriebs- und Dosiermodul nicht gemacht werden, kann das Modul insbesondere dem des Injektionsgeräts der Figur 1 entsprechen.

Das Antriebs- und Dosiermodul der Erfindung weist insbesondere eine Dosiersicherung auf, die eine Verstellung der eingestellten Produktdosis in der Auslöseposition der Fördereinrichtung verhindert. Die Dosiersicherung verhindert eine Verstellung der Produktdosis ferner auch während der Bewegung der Fördereinrichtung aus der Freigabeposition in die Auslöseposition, d. h. während der Rücksetzbewegung. Die Dosiersicherung wird durch einen Eingriff zwischen der Fördereinrichtung und der Dosiereinrichtung 8 gebildet, der während der Rücksetzbewegung und in der Auslöseposition der Fördereinrichtung Dosierbewegungen der Dosiereinrichtung 8 relativ zu der Fördereinrichtung blockiert und deshalb im Folgenden als Blockiereingriff bezeichnet wird.

Um die Dosiersicherung zu erhalten, weist die Fördereinrichtung zusätzlich zu den Gliedern der bekannten Fördereinrichtung 3-6 mehrere Eingriffsglieder 7a, im Ausführungsbeispiel zwei Eingriffsglieder 7a, auf, und die Dosiereinrichtung 8 ist mit mehreren Führungen 9 versehen. Die Führungen 9 sind an einer Mantelinnenfläche der Dosiereinrichtung 8 gebildet. Sie erstrecken sich je parallel zu der Achse R und sind um die Achse R gleichmäßig verteilt. Im Ausführungsbeispiel sind sie als von der Mantelinnenfläche vorragende Führungsstege gebildet.

Von den Eingriffsgliedern 7a ist in dem Längsschnitt der Figur 2 nur eines erkennbar. Das andere Eingriffsglied 7a entspricht dem eingezeichneten. Das Eingriffsglied 7a ist relativ zu dem Antriebsglied 5 und dem Betätigungselement 6 radial beweglich. Es ist mit dem Antriebsglied 5 in einem ersten Gelenk und mit dem Betätigungselement 6 in einem zweiten Gelenk verbunden. Das erste Gelenk ist ein einfaches Drehgelenk. Das Antriebsglied 5 bildet als Gelenkelement 14 des ersten Gelenks einen Zapfen, und das Eingriffsglied 7a bildet als Gelenkelement 21 eine Buchse; genauer gesagt bilden ein Halbzapfen und eine Halbbuchse die beiden Gelenkelemente 14 und 21. Die Drehachse dieses Gelenks 14, 21 weist quer zu der Achse R. Das zweite Gelenk ist ein Kurvengelenk. Das Eingriffsglied 7a bildet eine schlitzartige Führung 20 und das Betätigungselement 6 bildet ein Eingriffsglied 17 des zweiten Gelenks 17, 20. Das Kurvengelenk 17, 20 wandelt eine Axialbewegung, die das Betätigungselement 6 während der Rücksetzbewegung relativ zu dem Antriebsglied 5 ausführt, in eine Radialbewegung des Eingriffsglieds 7a um. Die Radialbewegung ist eine Schwenkbewegung um die von dem Gelenk 14, 21 gebildete Drehachse, indem der die Führung 20 bildende, von der Schwenkachse aus gesehen proximale Abschnitt des Eingriffsglieds 7a in den Blockiereingriff der radial gegenüberliegenden der Führungen 9 bewegt wird. Die diese Bewegung des Eingriffsglieds 7a bewirkende Bewegung, die das Betätigungselement 6 relativ zu dem Antriebsglied 5 ausführt, entspricht einem Leerhub der Fördereinrichtung 3-7, da diese Bewegung keine Rücksetzbewegung des Antriebsglieds 5 relativ zu der Kolbenstange 4 bewirkt. Es bewegen sich lediglich das Betätigungselement 6 axial in die proximale Richtung und das Eingriffsglied 7a, d. h. die beiden Eingriffsglieder 7a, zusätzlich in den Blockiereingriff. Die Anzahl der Führungen 9 und deren Verteilung um die Achse R ist so, dass in jeder der möglichen Dosierpositionen der Dosiereinrichtung 8 die Eingriffsglieder 7a in der Auslöseposition und während der Rücksetzbewegung des Betätigungselements 6 je im Eingriff mit wenigstens einer der Führungen 9 sind.

Aus dem in Figur 2 dargestellten Zustand des Moduls, in dem die Fördereinrichtung die Auslöseposition einnimmt, kann die eingestellte Produktdosis unmittelbar gefördert und dadurch ausgeschüttet werden. Es muss lediglich durch Druck auf das Betätigungselement 6 die Kolbenstange 4 in die distale Richtung bewegt werden. Die Kolbenstange 4 überträgt ihre Förderbewegung auf den Kolben 3, der für die Ausschüttung des Produkts deshalb die gleiche Förderbewegung ausführt. Bei seiner Bewegung aus der Auslöseposition drückt das Betätigungselement 6 gegen das nach außen in den Blockiereingriff bewegte Eingriffsglied 7a, das wiederum über die Gelenkverbindung 14, 21 das Antriebsglied 5 in die distale Richtung drückt. Das Antriebsglied 5 schließlich überträgt seine Axialbewegung mittels seiner in die Zahnreihen der Kolbenstange 4 eingreifenden Mitnehmer auf die Kolbenstange 4 und diese, wie bereits erwähnt, auf den Kolben 3. Diese gemeinsame Bewegung der gesamten Fördereinrichtung 3-7, wobei mit 7 die Eingriffsglieder 7a bezeichnet sein sollen, wird durch einen Förderanschlag 11, den das Gehäuse 1 bildet, und einen Förderanschlag 12, den das Antriebsglied 5 bildet, begrenzt, indem der Förderanschlag 12 in Kontakt mit dem Förderanschlag 11 gelangt. In der Anschlagposition der beiden Förderanschläge 11 und 12 und distaler Position des Betätigungselements 6 ist der Blockiereingriff zwischen der Fördereinrichtung 3-7 und der Dosiereinrichtung 8 gelöst oder zumindest soweit gelockert, dass die Dosiereinrichtung 8 Dosierbewegungen relativ zu der Fördereinrichtung 3-7 ausführen kann, weshalb diese Axialposition im Folgenden als Freigabeposition bezeichnet wird.

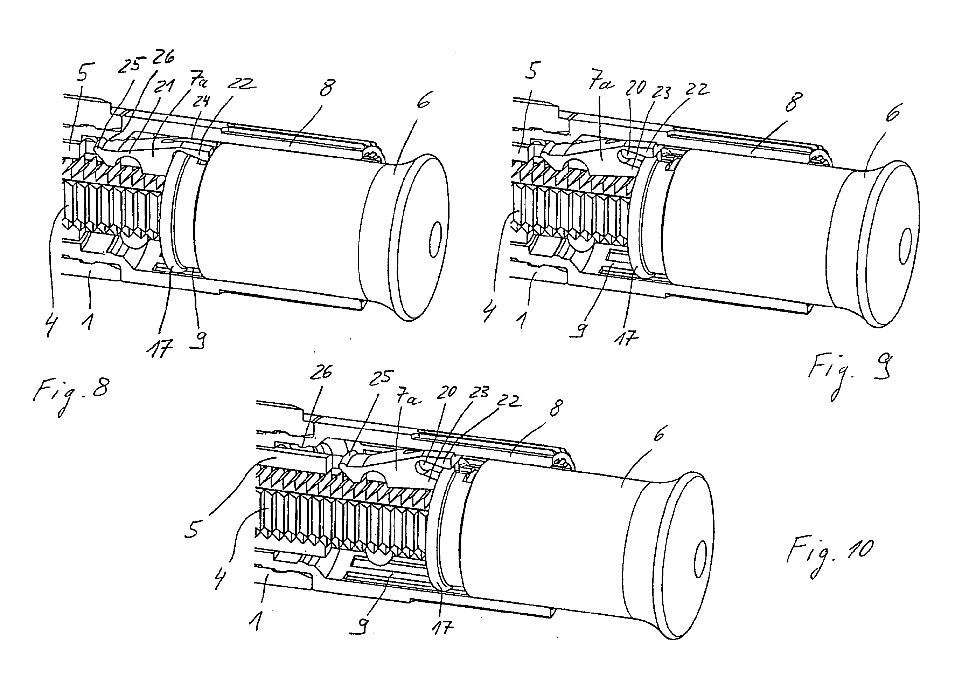

In Bezug auf die Dosiersicherung, insbesondere den Blockiereingriff, die Eingriffsglieder 7a und deren Zusammenwirken mit dem Betätigungselement 6, sei ergänzend stets auch auf die Figuren 8 bis 10 hingewiesen, die diesen Teil des Antriebs- und Dosiermoduls in dreidimensionaler Sicht zeigen.

Figur 10 zeigt die Dosiersicherung in dem in Figur 2 dargestellten Zustand. Aus Gründen der Übersichtlichkeit ist allerdings der mit den Eingriffsgliedern 7a verbundene, proximale Abschnitt des Antriebsglieds 5 nicht eingezeichnet. Gut zu erkennen ist insbesondere auch das Gelenkelement 17 des Betätigungselements 6. Das Gelenkelement 17, welches das Eingriffsglied des Kurvengelenks 17, 20 bildet, ist an dem Betätigungselement 6, nämlich an dessen distalen Ende, einstückig angeformt. Es bildet einen Hülsenabschnitt des Betätigungselements 6 mit einem wulstartig um die Achse R umlaufenden distalen Rand, der in die Führung 20 des Eingriffsglieds 7a und auch in die Führung 20 des anderen, bezüglich der Achse R diametral gegenüberliegenden Eingriffsglieds 7a eingreift. Die Führung 20 ist axial erstreckt und weist von proximal nach distal geneigt zu der Achse R nach außen. Die Führung 20 umfasst eine radial äußere Führung 22 und der äußeren Führung 22 gegenüberliegend eine radial innere Führung 23. Das Gelenkelement 17 des Betätigungselements 6 wirkt mit beiden Führungen 22 und 23 zusammen. Bei der Rücksetzbewegung des Betätigungselements drückt das Gelenkelement 17 gegen die äußere Führung 22 und dadurch das Eingriffselement 7a wegen der Neigung der äußeren Führung 22 in den Blockiereingriff. Bei der Bewegung in die distale Richtung drückt das Gelenkelement 17 gegen die innere Führung 23 und dadurch das Eingriffsglied 7a wegen der Neigung der inneren Führung 23 aus dem Blockiereingriff. An ihrem distalen Ende ist die mittels der Führungen 22 und 23 gebildete Führung 20 erweitert, um in der Freigabeposition der Fördereinrichtung 3-7 den wulstartigen Rand des Gelenkelements 17 aufnehmen zu können. Die schlitzartige Führung 20 ist in die proximale Richtung offen, was die Herstellung der Gelenkverbindung 17, 20 erleichtert.

Das Eingriffsglied 7a ist in seinem proximalen Bereich an seiner radialen Außenfläche mit einer axialen Eingriffsnut 24 versehen, die am besten in Figur 8 zu erkennen ist. Im Blockiereingriff, den die Figuren 9 und 10 zeigen, greift eine der Führungen 9 in die Nut 24 des Eingriffsglieds 7a, so dass die Dosiereinrichtung 8 relativ zu dem Antriebsglied 5 keine Dosierbewegungen quer zu der eingreifenden Führung 9 aber Axialbewegungen ungehindert ausführen kann. Diesbezüglich sei noch angemerkt, dass die Eingriffsglieder 7a je um die Achse R verdrehgesichert mit dem Antriebsglied 5 verbunden sind.

Das Eingriffsglied 7a bildet ein Koppelglied, das bei einer Betätigung des Betätigungselements 6 dessen Bewegung auf das Antriebsglied 5 überträgt. Bei Druckbetätigung des Betätigungselements 6 wirkt es als Anschlag für das Betätigungselement 6. Für die Übertragung einer Rücksetzbewegung des Betätigungselements 6 hintergreift das Eingriffsglied 7a das Betätigungselement 6. Für das Hintergreifen bildet das Eingriffsglied 7a ein Koppelelement 28, im Ausführungsbeispiel in der Art eines Hakens. Das Betätigungselement 6 ist mit einer von dem Koppelelement 28 hintergreifbaren Schulter versehen, die im Ausführungsbeispiel durch eine Ausnehmung an einer Mantelinnenfläche oder durch einen Durchbruch im Mantel des Betätigungselements 6 gebildet wird. Das Koppelelement 28 ist an einem die innere Führung 23 bildenden Arm des Eingriffsglieds 7a gebildet. Es ragt dort nach radial auswärts vor. Durch die Schwenkbewegung des Eingriffsglieds 7a beim Herstellen des Blockiereingriffs wird das Koppelelement 28 in den Hintergriff mit der Schulter des Betätigungselements 6 geschwenkt. Das Eingriffsglied 7a bildet somit nicht nur das Eingriffsglied für den Blockiereingriff, sondern dient bei der Förderung des Produkts als Druckglied und bei der Rücksetzung der Fördereinrichtung 3-7 als Zugglied.

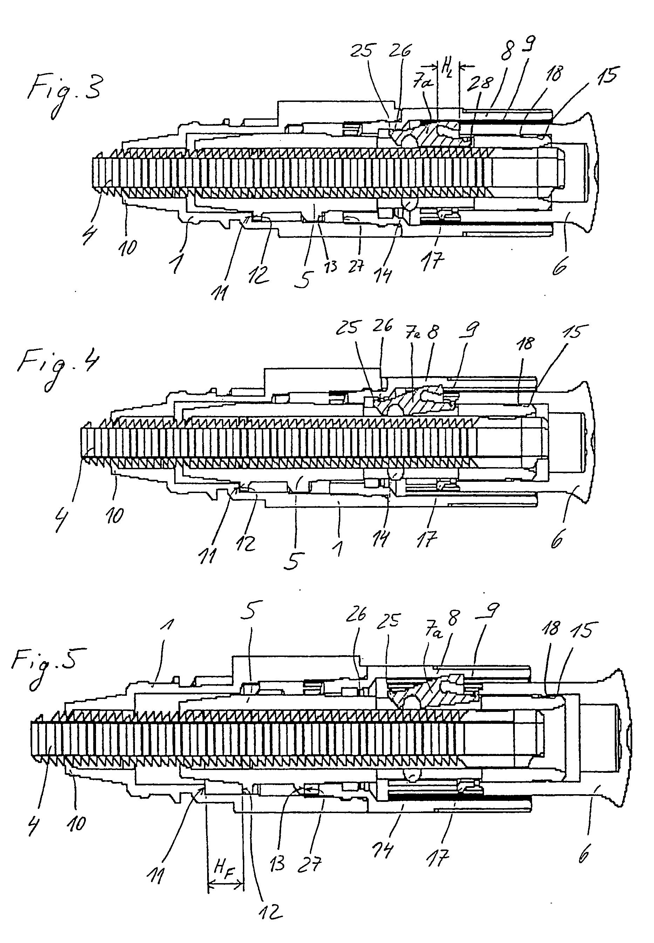

Nachfolgend wird die Funktionsweise eines Injektionsgeräts, das mit dem erfindungsgemäßen Antriebs- und Dosiermodul ausgerüstet ist, anhand einer in den Figuren 3 bis 7 dargestellten Ablaufsequenz beschrieben. Ergänzend sei stets auf die Figuren 1 und 2 und insbesondere auch auf die Figuren 8 bis 10 verwiesen, die das Zusammenwirken der einzelnen Glieder detaillierter erkennen lassen. In den Figuren 3 bis 7 ist jeweils nur das Antriebs- und Dosiermodul dargestellt, der vordere Abschnitt des Gehäuses 1, der das Reservoir 2 mit dem Kolben 3 aufnimmt, kann jedoch ohne Weiteres hinzugedacht werden.

Figur 3 zeigt das Antriebs- und Dosiermodul in einem Zustand, in dem die Fördereinrichtung 3-7 relativ zu dem Gehäuse 1 und der Dosiereinrichtung 8 die Freigabeposition einnimmt. In der Freigabeposition wird die zu verabreichende Produktdosis mittels der Dosiereinrichtung 8 eingestellt. Für die Einstellung wird die Dosiereinrichtung 8 um die Achse R relativ zu dem Gehäuse 1 und insbesondere zum Antriebsglied 5 in die der einzustellenden Produktdosis entsprechende Dosierposition bewegt. Aufgrund des bei der Dosierbewegung wirksamen Rastmechanismus vernimmt der Verwender bei dem Einstellen ein Klickgeräusch. Ferner spürt er das Rasten von einer Dosierposition in die andere. Zusätzlich ist eine nicht dargestellte optische Anzeige der Produktdosis vorgesehen. Wenn die gewünschte Produktdosis eingestellt ist, wird das Injektionsgerät "geladen", indem die Fördereinrichtung 3-7 in die distale Richtung bis in die Auslöseposition bewegt wird. Diese Rücksetzbewegung wird durch Ziehen an dem Betätigungselement 6 bewirkt.

Die Rücksetzbewegung, wie auch die Bewegung der Fördereinrichtung 3-7 in die Freigabeposition, ist in zwei Phasen unterteilt. In einer ersten Phase der Rücksetzbewegung wird das Betätigungselement 6 relativ zu dem Gehäuse 1, der Dosiereinrichtung 8 und insbesondere auch relativ zu dem Antriebsglied 5 in die proximale Richtung bewegt. Der Hub, d. h. die Weglänge, dieser Leerbewegung ist mit H_{L} bezeichnet. Die erste Phase der Rücksetzbewegung ist beendet, wenn das Betätigungselement 6 in Anschlag mit dem Koppelelement 28, d. h. in den Hintergriff, gelangt.

In den Figuren 2 bis 7 ist eine andere Möglichkeit der Begrenzung der Leerbewegung angedeutet, die bei der weiteren Rücksetzbewegung zusätzlich oder alternativ zu dem im Ausführungsbeispiel als Zugglied dienenden Eingriffsglied 7a die Mitnahme bewirken kann. Ein hierfür vorgesehener Anschlag des Antriebsglieds 5 ist an dessen Mantelaußenfläche gebildet und mit dem Bezugszeichen 15 versehen. Das Betätigungselement 6 bildet einen Gegenanschlag 18 als Anschlagring oder Anschlagnocken, der von der umgebenden Mantelfläche des Betätigungselements 6 nach radial innen vorragt.

Das Betätigungselement 6 ist über das in der Aufweitung der Führung 20 aufgenommene Gelenkelement 17 und ein Hakenelement 25 des Eingriffsglieds 7a an der Dosiereinrichtung 8 verhakt, so dass es nicht bereits unter seinem Eigengewicht in die proximale Richtung rutschen kann, sondern eine gewisse Zugkraft aufgebracht werden muss, um die Rücksetzbewegung zu bewirken. Das Hakenelement 25 ist an dem Eingriffsglied 7a distal von dessen Schwenkachse gebildet. In der Freigabeposition hintergreift das Eingriffsglied 7a mit seinem Hakenelement 25 ein Hakenelement 26 der Dosiereinrichtung 8.

Figur 4 zeigt das Antriebs- und Dosiermodul nach Ausführung der ersten Phase (Leerbewegung) der Rücksetzbewegung, aber noch vor Einsetzen der zweiten Phase. Der Mitnehmeranschlag 18 ist gerade in Kontakt mit dem Mitnehmeranschlag 15 gelangt. Bei einem weiteren Zurückziehen des Betätigungselements 6 wird ab jetzt das Antriebsglied 5 durch den Kontakt der Mitnehmeranschläge 15 und 18 in die proximale Richtung mitgenommen. Die Mitnehmer des Antriebsglieds 5 gleiten dabei über die Zähne der Kolbenstange 4, die wegen des Eingriffs der Rückhalteeinrichtung 10 an einer Bewegung in die proximale Richtung gehindert ist.

Während der ersten Phase der Rücksetzbewegung gleitet das Gelenkelement 17 des Betätigungselements 6 entlang der äußeren Führung 22 an dem Eingriffsglied 7a ab. Wegen des in die proximale Richtung nach einwärts geneigten Verlaufs der äußeren Führung 22 kippt das Eingriffsglied 7a um seine in der Gelenkverbindung 14, 21 gebildete Schwenkachse in den Blockiereingriff mit der gegenüberliegenden der Führungen 9. Die Kippbewegung und die damit einhergehende Radialbewegung des proximalen Abschnitts des Eingriffsglieds 7a ist im Vergleich der Figuren 3 und 4 und insbesondere im Vergleich der Figuren 8 und 9, die mit den Figuren 3 und 4 korrespondieren, zu erkennen. Die Eingriffsglieder 7a sind je in sich steife Körper. Mit dem Kippen des proximal von der Schwenkachse gelegenen Eingriffsteils des Eingriffsglieds 7a in den Blockiereingriff kippt dessen distal von der Schwenkachse gelegenes Hakenelement 25 aus der Verhakung mit der Dosiereinrichtung 8, so dass das Antriebsglied 5 relativ zu der Dosiereinrichtung 8 und dem Gehäuse 1 in die proximale Richtung bewegt werden kann.

Durch die Bewegung des Gelenkelements 17 entlang der äußeren Führung 22 wird das Eingriffsglied 7a soweit in den Blockiereingriff bewegt, dass es mit einer proximalen Anschlagfläche vor eine distale Anschlagfläche des Betätigungselements 6 zu liegen kommt. Über den derart gebildeten Anschlagkontakt drückt das Betätigungselement 6 im Falle einer Bewegung in die proximale Richtung gegen das Eingriffsglied 7a und bewegt somit über das Eingriffsglied 7a das Antriebsglied 5 ebenfalls in die proximale Richtung.

Aus dem in Figur 4 gezeigten Zustand wird durch weiteres Ziehen an dem Betätigungselement 6 die Fördereinrichtung 3-7 in die Auslöseposition bewegt. In dieser zweiten Phase der Rücksetzbewegung nimmt das Betätigungselement 6 über die Kopplung mittels des Koppelelements 28 oder/und durch die Mitnehmeranschläge 15 und 18 das Antriebsglied 5 mit. Von einer kurzen Anfangsphase abgesehen, in der das Eingriffsglied 7a in den Blockiereingriff bewegt wird, verhindert der Blockiereingriff während der gesamten Rücksetzbewegung und insbesondere auch in der Auslöseposition jegliche Dosierbewegung der Dosiereinrichtung 8.

Um auch in der Anfangsphase der Rücksetzbewegung die Sicherheit vor Fehldosierungen zu erhöhen, kann das Eingriffsglied 7a bereits in der Freigabeposition der Fördereinrichtung 3-7 in die gegenüberliegende der Führungen 9 eingreifen, allerdings nicht in einem Blockiereingriff, sondern in einem lösbaren Rasteingriff. Dieser Rasteingriff kann gleichzeitig auch den Rasteingriff für die Dosiereinrichtung 8 bei deren Dosierbewegung bilden. Das Eingriffsglied 7a wäre in solch einer Ausbildung nicht wie im Ausführungsbeispiel in der Freigabeposition der Fördereinrichtung 3-7 vollkommen frei von den Führungen 9, sondern würde weniger tief als im Blockiereingriff in eine oder mehrere der Führungen 9 eingreifen. Das Eingriffsglied 7a und/oder die Führungen 9 wären entsprechend zu formen, beispielsweise durch Abrunden in einem radial äußeren Bereich, um zum Einen den lösbaren Rasteingriff und zum Anderen den unlösbaren Blockiereingriff bilden zu können.

Figur 5 zeigt das Antriebs- und Dosiermodul in einem Zustand, in dem die Fördereinrichtung 3-7 die Auslöseposition einnimmt. In der Auslöseposition sind die Dosieranschläge 13 und 27 des Antriebsglieds 5 und der Dosiereinrichtung 8 gegeneinander auf Anschlag. Für die Betätigung der Fördereinrichtung 3-7 und die dadurch bewirkte Förderung und Ausschüttung der eingestellten Produktdosis wird das Betätigungselement 6 mit einer Druckkraft in die proximale Richtung beaufschlagt. Das Betätigungselement 6 drückt in die proximale Richtung gegen das Eingriffsglied 7a und über das Eingriffsglied 7a und dessen Gelenkverbindung 14, 21 mit dem Antriebsglied 5 auch gegen das Antriebsglied 5. Das Betätigungselement 6, die Eingriffsglieder 7a und das Antriebsglied 5 werden daher in die proximale Richtung bewegt. Wegen des Mitnahmeeingriffs zwischen dem Antriebsglied 5 und der Kolbenstange 4 werden die Kolbenstange 4 gemeinsam mit dem Antriebsglied 5 und der Kolben 3 gemeinsam mit der Kolbenstange 4 in die proximale Richtung bewegt. Dieser Förderhub H_{F} der Fördereinrichtung 3-7 wird durch den Anschlagkontakt des Anschlagpaars 11 und 12 begrenzt. Der in der Freigabeposition der Fördereinrichtung 3-7 (Figur 3) eingestellte axiale Abstand zwischen den Förderanschlägen 11 und 12 entspricht dem Förderhub H_{F}, d. h. der Weglänge, die der Kolben 3 für die Ausschüttung der eingestellten Produktdosis zurücklegt.

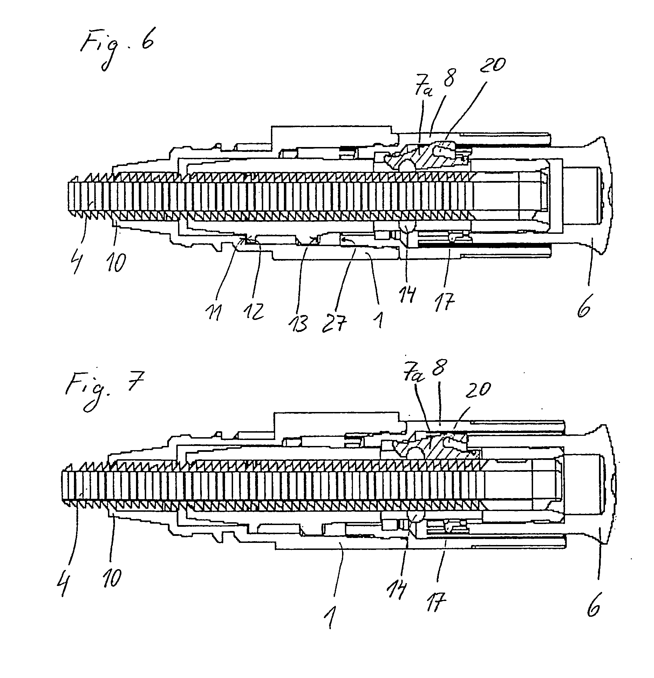

Figur 6 zeigt das Antriebs- und Dosiermodul nach der vollständigen Ausführung der Förderbewegung. Das Anschlagpaar 11 und 12 ist auf Anschlagkontakt. Das Betätigungselement 6 nimmt allerdings noch nicht seine distale Endposition relativ zu dem Antriebsglied 5 ein, vielmehr nimmt es noch immer relativ zu dem Antriebsglied 5 die Axialposition ein, die es während der Förderbewegung innehat. Dementsprechend sind die Fördereinrichtung 3-7 und die Dosiereinrichtung 8 noch im Blockiereingriff. Der Übergang aus diesem Zustand in die sich anschließende Phase der Leerbewegung des Betätigungselements ist fließend. Dabei gleitet zunächst das Gelenkelement 17 an der inneren Führung 23 (beispielsweise Figur 10) des Eingriffsglieds 7a ab, wodurch das Eingriffsglied 7a nach radial einwärts aus dem Blockiereingriff und in seinem distalen Bereich nach radial auswärts in die Verhakung mit der Dosiereinrichtung 8 kippt.

Figur 7 zeigt das Antriebs- und Dosiermodul mit der wieder in der Freigabeposition befindlichen Fördereinrichtung 3-7. Der einzige Unterschied zu dem in Figur 3 gezeigten Zustand ist, dass die Kolbenstange 4 relativ zu dem Antriebsglied 5 um die axiale Weglänge H_{F} der Förderbewegung in die distale Richtung verschoben ist. Das Gerät ist nun bereit für eine erneute Dosisauswahl oder einen erneuten Ladevorgang unter Beibehaltung der eingestellten Dosis.

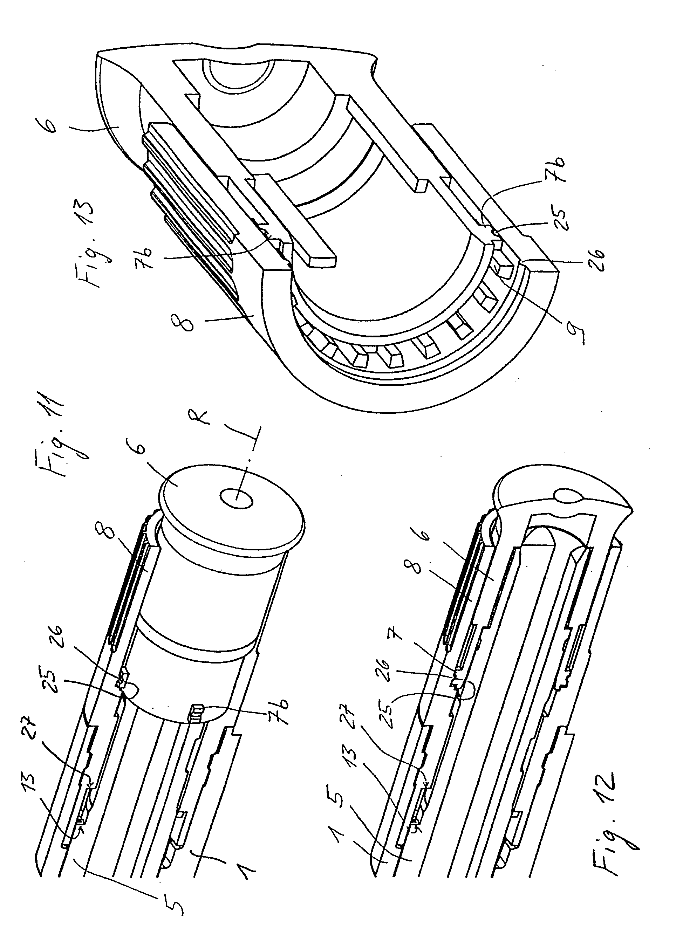

Die Figuren 11 bis 13 zeigen einen proximalen Endabschnitt eines modifizierten Antriebs- und Dosiermoduls, das anstatt des Moduls des ersten Ausführungsbeispiels das Antriebs- und Dosiermodul des Injektionsgeräts der Figur 1 ersetzen kann. Soweit die Teile des modifizierten Moduls die gleichen Funktionen wie vorstehend beschrieben ausführen, sind die gleichen Bezugszeichen gewählt und sei auf die vorstehenden Ausführungen verwiesen. Im Unterschied zu dem ersten Ausführungsbeispiel sind die Eingriffsglieder der Fördereinrichtung 3-7 an dem Betätigungselement 6 starr angeordnet. Im Ausführungsbeispiel sind sie mit dem Betätigungselement 6 in einem Stück geformt. Zwecks Unterscheidung zu dem ersten Ausführungsbeispiel sind die Eingriffsglieder des modifizierten Moduls mit dem Bezugszeichen 7b versehen. Die Eingriffsglieder 7b ragen in einem distalen Endabschnitt von der Mantelaußenfläche des Betätigungselements 6 ab. Sie sind als Eingriffsnocken gebildet.

Die Dosiereinrichtung 8, die das Betätigungselement 6 und das Antriebsglied 5 wieder um die Achse R umgibt, ist in ihrem das Betätigungselement 6 umgebenden Abschnitt an ihrer Mantelinnenfläche wieder mit Führungen 9 in Form von geraden, parallel zu der Achse R erstreckten Führungsstegen 9 versehen, zwischen denen die Eingriffsglieder 7b im Blockiereingriff eingreifen und an denen sie bei der Rücksetzbewegung entlang fahren. Was den Blockiereingriff als solchen anbetrifft, besteht kein Unterschied zum Antriebs- und Dosiermodul des ersten Ausführungsbeispiels. Allerdings kommen die Eingriffsglieder 7b in der Freigabeposition der Fördereinrichtung 3-7 nicht radial unter den Führungen 9, sondern auf radial gleicher Höhe proximal vor die Führungen 9 zu liegen. In der Freigabeposition bilden die Eingriffsglieder 7b ferner Rastelemente 25 für einen Rasteingriff mit einem Rastelement 26 der Dosiereinrichtung 8. Die Rastelemente 25 und 26 übernehmen die Funktion der Hakenelemente 25 und 26 des ersten Ausführungsbeispiels. Im Ausführungsbeispiel sind die Rastelemente 25 Vertiefungen, von denen je eine pro Eingriffsglied 7b gebildet ist. Das Rastelement 26 der Dosiereinrichtung 8 ist ein umlaufender Ringsteg, der in der Freigabeposition der Fördereinrichtung 3-7 in die Vertiefungen 25 der Eingriffsglieder 7b eingreift. Dieser Rasteingriff ist wie die Verhakung des ersten Ausführungsbeispiels durch Ziehen an dem Betätigungselement 6 lösbar.

Die Figuren 11 und 12 zeigen das Antriebs- und Dosiermodul des zweiten Ausführungsbeispiels je mit in der Freigabeposition befindlicher Fördereinrichtung 3-7, wobei in Figur 11 das Betätigungselement 6 in einer perspektivischen Ansicht und in Figur 12 perspektivisch in einem Längsschnitt dargestellt ist. Figur 13 zeigt das modifizierte Modul während der Rücksetzbewegung der Fördereinrichtung 3-7, d. h. nach dem Lösen des Rasteingriffs der Rastelemente 25 und 26, mit bereits bestehendem Blockiereingriff der Eingriffsglieder 7b je zwischen zwei benachbarten Führungen 9.

### Bezugszeichen:

- 1: Gehäuse
- 2: Reservoir
- 3: Förderglied, Kolben
- 4: Abtriebsglied, Kolbenstange
- 5: Antriebsglied
- 6: Betätigungselement
- 7a: Eingriffsglied
- 7b: Eingriffsglied
- 8: Dosiereinrichtung, Dosierglied
- 9: Führung
- 10: Rückhalteeinrichtung
- 11: Förderanschlag
- 12: Förderanschlag
- 13: Dosieranschlag
- 14: Gelenkelement, Zapfen
- 15: Mitnehmeranschlag
- 16: -
- 17: Gelenkelement, Eingriffsglied
- 18: Mitnehmeranschlag
- 19: -
- 20: Gelenkelement, Führung
- 21: Gelenkelement, Buchse
- 22: äußere Führung
- 23: innere Führung
- 24: Eingriffsnut
- 25: Rastelement, Haken
- 26: Rastelement, Haken
- 27: Dosieranschlag
- 28: Koppelelement

- N: Nadel
- R: Rotationsachse

## Patentansprüche

1. Vorrichtung zur Verabreichung eines injizierbaren Produkts, umfassend:
a) ein Gehäuse (1) mit einem Reservoir (2) für das Produkt,
b) eine Fördereinrichtung (3-7), die aus einer Freigabeposition in eine Auslöseposition bewegbar ist und aus der Auslöseposition eine Förderbewegung ausführt, durch die eine eingestellte Produktdosis aus dem Reservoir (2) ausgeschüttet wird,
c) und eine Dosiereinrichtung (8), die für die Einstellung der Produktdosis eine Dosierbewegung in je durch Rasteingriff vorgegebene Dosierpositionen ausführen kann, wenn die Fördereinrichtung (3-7) die Freigabeposition einnimmt, **dadurch gekennzeichnet, daß**
d) die Fördereinrichtung (3-7) in der Auslöseposition und bei der Bewegung in die Auslöseposition mit der Dosiereinrichtung (8) über einen Blockiereingriff, der die Dosiereinrichtung (8) in der jeweils eingestellten Dosierposition gegen Dosierbewegungen blockiert, gekoppelt ist.

2. Vorrichtung nach dem vorhergehenden Anspruch, **dadurch gekennzeichnet, dass** die Dosiereinrichtung (8) einen durch die Dosierbewegung verstellbaren Dosieranschlag (27) bildet, der die Bewegung der Fördereinrichtung (3-7) aus der Freigabeposition in die Auslöseposition begrenzt und dadurch eine Weglänge (H_{F}) der Förderbewegung bestimmt.

3. Vorrichtung nach dem vorhergehenden Anspruch, **dadurch gekennzeichnet, dass** mehrere diskrete Anschläge gemeinsam den verstellbaren Dosieranschlag bilden, dass pro Dosierposition nur einer oder eine Gruppe der Anschläge die Bewegung der Fördereinrichtung begrenzt und dass für die einstellbaren Produktdosen, die den Dosierpositionen entsprechen, je wenigstens einer der Anschläge vorgesehen ist.

4. Vorrichtung nach Anspruch 2, **dadurch gekennzeichnet, dass** der Dosieranschlag (27) sich kontinuierlich mit einer Neigung zu der Richtung der Förderbewegung der Fördereinrichtung (3-7) erstreckt.

5. Vorrichtung nach einem der vorhergehenden Ansprüche, **dadurch gekennzeichnet, dass** in den Dosierpositionen je eines aus Fördereinrichtung (3-7) und Dosiereinrichtung (8) eine Führung (9) und das andere ein Eingriffsglied (7a; 7b) bildet und dass das Eingriffsglied (7a; 7b) in dem Blockiereingriff entlang der Führung (9) in Richtung der in die Auslöseposition stattfindenden Bewegung der Fördereinrichtung (3-7) geführt wird.

6. Vorrichtung nach dem vorhergehenden Anspruch, **dadurch gekennzeichnet, dass** die Führung (9) und das Eingriffsglied (7a; 7b) in der Freigabeposition der Fördereinrichtung (3-7) voneinander frei sind.

7. Vorrichtung nach Anspruch 5, **dadurch gekennzeichnet, dass** die Führung (9) und das Eingriffsglied (7a) in der Freigabeposition der Fördereinrichtung (3-7) miteinander in einem lösbaren Rasteingriff sind.

8. Vorrichtung nach dem vorhergehenden Anspruch, **dadurch gekennzeichnet, dass** der Rasteingriff der Führung (9) und des Eingriffsglieds (7a) sich bei der in die Auslöseposition stattfindenden Bewegung der Fördereinrichtung (3-7) zu dem Blockiereingriff verstärkt.

9. Vorrichtung nach einem der vier vorhergehenden Ansprüche, **dadurch gekennzeichnet, dass** die Dosierbewegung eine Drehbewegung der Dosiereinrichtung (8) relativ zu der Fördereinrichtung (3-7) um eine Rotationsachse (R) umfasst, die Förderbewegung eine Bewegung der Fördereinrichtung (3-7) relativ zu der Dosiereinrichtung (8) entlang der Rotationsachse (R) umfasst, eines aus Fördereinrichtung (3-7) und Dosiereinrichtung (8) das andere wenigstens teilweise umgibt und dass die Führung (9) und das Eingriffsglied (7a; 7b) an einander zugewandt gegenüberliegenden Mantelflächen der Fördereinrichtung (3-7) und der Dosiereinrichtung (8) vorgesehen sind.

10. Vorrichtung nach dem vorhergehenden Anspruch, **dadurch gekennzeichnet, dass** wenigstens eines aus Führung (9) und Eingriffsglied (7a) relativ zu der Mantelfläche, an der sie oder es vorgesehen ist, radial bewegbar angeordnet ist und durch die in die Auslöseposition stattfindende Bewegung der Fördereinrichtung (3-7) auf die gegenüberliegende Mantelfläche zu und dadurch in den Blockiereingriff bewegt wird.

11. Vorrichtung nach einem der Ansprüche 2 bis 4 in Kombination mit einem der Ansprüche 5 bis 10, **dadurch gekennzeichnet, dass** die Dosiereinrichtung (8) eines aus Eingriffsglied (7a; 7b) und Führung (9) zusätzlich zu dem Dosieranschlag (27) bildet.

12. Vorrichtung nach einem der sieben vorhergehenden Ansprüche, **dadurch gekennzeichnet, dass** eines aus Eingriffsglied (7a) und Führung (9) mittels eines Gelenks (14, 21) beweglich mit der Fördereinrichtung (3-7) verbunden ist.

13. Vorrichtung nach einem der acht vorhergehenden Ansprüche, **dadurch gekennzeichnet, dass** die Fördereinrichtung (3-7) ein manuell betätigbares Betätigungselement (6) umfasst, dessen Betätigung die Förderbewegung bewirkt, und dass das Betätigungselement (6) und eines aus Eingriffsglied (7b) und Führung (9) zusammen in einem Stück gebildet sind.

14. Vorrichtung nach einem der Ansprüche 5 bis 12, **dadurch gekennzeichnet, dass** die Fördereinrichtung (3-7) ein manuell betätigbares Betätigungselement (6) umfasst, dessen Betätigung die Förderbewegung bewirkt, und dass das Betätigungselement (6) mit einem aus Eingriffsglied (7a) und Führung (9) über ein Gelenk (17, 20) verbunden ist, das eine bei Betätigung ausgeführte Bewegung des Betätigungselements (6) in eine Bewegung des einen aus Eingriffsglied (7a) und Führung (9), das über das Gelenk (17, 20) mit dem Betätigungselement (6) verbunden ist, in den Blockiereingriff umwandelt.

15. Vorrichtung nach dem vorhergehenden Anspruch, **dadurch gekennzeichnet, dass** das Gelenk (17, 20) ein Kurvengelenk ist und ein Gelenkelement (17) des Betätigungselements (6) und ein Gelenkelement (20) des einen aus Eingriffsglied (7a) und Führung (9) in dem Gelenk (17, 20) vorzugsweise in und gegen die Richtung der Förderbewegung der Fördereinrichtung (3-7) relativ zueinander bewegbar sind.

16. Vorrichtung nach einem der zwei vorhergehenden Ansprüche, **dadurch gekennzeichnet, dass** das Betätigungselement (6) über das eine aus Eingriffsglied (7a) und Führung (9), das mit dem Betätigungselement (6) über das Gelenk (17, 20) verbunden ist, die Förderbewegung der Fördereinrichtung (3-7) und/oder deren Bewegung in die Auslöseposition bewirkt.

17. Vorrichtung nach einem der vorhergehenden Ansprüche, **dadurch gekennzeichnet, dass** die Fördereinrichtung (3-7) ein manuell betätigbares Betätigungselement (6) umfasst, dessen Betätigung die Förderbewegung bewirkt, und dass eine Weglänge (H_{F}+H_{L}) einer von dem Betätigungselement (6) bei Betätigung ausführbaren Bewegung größer ist als die der vollständigen Ausschüttung der eingestellten Produktdosis entsprechende Weglänge (H_{F}) der Förderbewegung.

18. Vorrichtung nach dem vorhergehenden Anspruch, **dadurch gekennzeichnet, dass** die Fördereinrichtung (3-7) wenigstens ein in dem Reservoir (2) angeordnetes Förderglied (3) aufweist, das die Förderbewegung ausführt, und dass das Betätigungselement (6) mit dem Förderglied (3) so gekoppelt ist, dass es über einen Teil (H_{F}) der Weglänge (H_{F}+H_{L}) seiner Bewegung das Förderglied (3) mitnimmt und den anderen Teil (H_{L}) der Weglänge seiner Bewegung (H_{F}+H_{L}) ohne das Förderglied (3) zurücklegt.

19. Vorrichtung nach einem der vorhergehenden Ansprüche, **dadurch gekennzeichnet, dass** die Fördereinrichtung (3-7) ein manuell betätigbares Betätigungselement (6) umfasst, dessen Betätigung die Förderbewegung bewirkt, und dass das Betätigungselement (6) mittels einer lösbaren Rastverbindung (25, 26) an einem aus Dosiereinrichtung (8) und Gehäuse (1) gegen die Richtung der Förderbewegung festgelegt ist, wenn die Fördereinrichtung (3-7) die Freigabeposition einnimmt.

20. Vorrichtung nach einem der vorhergehenden Ansprüche, **dadurch gekennzeichnet, dass** der Blockiereingriff sich automatisch löst, wenn die Fördereinrichtung (3-7) in die Freigabeposition bewegt wird.

21. Vorrichtung nach einem der vorhergehenden Ansprüche, **dadurch gekennzeichnet, dass** die Fördereinrichtung (3-7) sich durch die Förderbewegung in die Freigabeposition bewegt.

22. Vorrichtung nach einem der vorhergehenden Ansprüche, **dadurch gekennzeichnet, dass** die Dosiereinrichtung (8) die Dosierbewegung wahlfrei in Richtung einer Vergrößerung und in Richtung einer Verkleinerung der Produktdosis ausführen kann.

## Claims

1. Device for administering an injectable product, comprising:
a) a casing (1) with a reservoir (2) for the product,
b) a feed device (3-7) which is movable from an unblocking position to a release position and from the release position executes a feed movement by which a set product dose is dispensed from the reservoir (2),
c) and a dosing device (8) which, for setting the product dose, can execute a dosing movement to dosing positions, each predefined by latching engagement, when the feed device (3-7) adopts the unblocking position,
**characterised in that**
d) the feed device (3-7), in the release position and during its movement to the release position, is coupled to the dosing device (8) via a locking engagement which locks the dosing device (8) against dosing movements in the particular dosing position set.

2. Device according to the preceding claim, **characterised in that** the dosing device (8) forms a dosing stop (27) which is adjustable through the dosing movement, and which limits the movement of the feed device (3-7) from the unblocking position to the release position and thereby determines a displacement distance (H_{F}) of the feed movement.

3. Device according to the preceding claim, **characterised in that** a plurality of discrete stops jointly form the adjustable dosing stop, **in that** only one or a group of the stops limit/s the movement of the feed device per dosing position, and **in that** at least one of the stops is provided for each of the adjustable product doses which correspond to the dosing positions.

4. Device according to claim 2, **characterised in that** the dosing stop (27) extends continuously with an inclination with respect to the direction of the feed movement of the feed device (3-7).

5. Device according to any one of the preceding claims, **characterised in that** in the dosing positions either the feed device (3-7) or the dosing device (8) forms a guide (9) while the other forms an engagement member (7a; 7b), and **in that** the engagement member (7a; 7b), when in locking engagement, is guided along the guide (9) in the direction of the movement of the feed device (3-7) taking place to the release position.

6. Device according to the preceding claim, **characterised in that** the guide (9) and the engagement member (7a; 7b) are free from one another in the unblocking position of the feed device (3-7).

7. Device according to claim 5, **characterised in that** the guide (9) and the engagement member (7a) are in releasable latching engagement with one another in the unblocking position of the feed device (3-7).

8. Device according to the preceding claim, **characterised in that** the latching engagement of the guide (9) and the engagement member (7a) is strengthened to become the locking engagement during the movement of the feed device (3-7) taking place to the release position.

9. Device according to any one of the four preceding claims, **characterised in that** the dosing movement includes a rotary movement of the dosing device (8) relative to the feed device (3-7) about an axis of rotation (R), the feed movement includes a movement of the feed device (3-7) relative to the dosing device (8) along the axis of rotation (R), either the feed device (3-7) or the dosing device (8) at least partially surrounding the other, and **in that** the guide (9) and the engagement member (7a; 7b) are provided on opposite lateral faces of the feed device (3-7) and of the dosing device (8) which face towards one another.

10. Device according to the preceding claim, **characterised in that** either the guide (9) or the engagement member (7a) or both is/are arranged radially movably relative to the lateral surface on which it/they is/are provided and is/are moved, by the movement of the feed device (3-7) taking place to the release position, towards the opposite lateral surface and thereby is/are moved into locking engagement.

11. Device according to any one of claims 2 to 4 in combination with any one of claims 5 to 10, **characterised in that** either the engagement member (7a; 7b) or the guide (9) forms the dosing device (8) additionally to the dosing stop (27).

12. Device according to any one of the seven preceding claims, **characterised in that** either the engagement member (7a) or the guide (9) is connected to the feed device (3-7) by means of a joint (14, 21).

13. Device according to any one of the eight preceding claims, **characterised in that** the feed device (3-7) includes a manually operable actuating element (6), actuation of which effects the feed movement, and **in that** the actuating element (6) is formed in one piece with either the engagement member (7b) or the guide (9).

14. Device according to any one of claims 5 to 12, **characterised in that** the feed device (3-7) includes a manually operable actuating element (6), actuation of which effects the feed movement, and **in that** the actuating element (6) is connected to either the engagement member (7a) or the guide (9) via a joint (17, 20) which converts a movement executed by the actuating element (6) upon actuation thereof into a movement of either the engagement member (7a) or the guide (9), whichever is connected via the joint (17, 20) to the actuating element (6), into locking engagement.

15. Device according to the preceding claim, **characterised in that** the joint (17, 20) is a cam joint and a joint element (17) of the actuating element (6) and a joint element (20) of either the engagement member (7a) or the guide (9) are movable relative to one another in the joint (17, 20) preferably in and against the direction of the feed movement of the feed device (3-7).

16. Device according to either of the two preceding claims, **characterised in that** the actuating element (6) effects the feed movement of the feed device (3-7) and/or its movement to the release position via either the engagement member (7a) or the guide (9), whichever is connected to the actuating element (6) via the joint (17, 20).

17. Device according to any one of the preceding claims, **characterised in that** the feed device (3-7) includes a manually operable actuating element (6), actuation of which effects the feed movement, and **in that** a displacement distance (H_{F}+H_{L}) of a movement executed by the actuating element (6) upon actuation thereof is greater than the displacement distance (H_{F}) of the feed movement corresponding to complete dispensing of the set product dose.

18. Device according to the preceding claim, **characterised in that** the feed device (3-7) includes at least one feed member (3) which is arranged in the reservoir (2) and executes the feed movement, and **in that** the actuating element (6) is coupled to the feed member (3) in such a way that it entrains the feed member (3) over a part (H_{F}) of the displacement distance (H_{F}+H_{L}) of its movement and travels the other part (H_{L}) of the displacement distance of its movement (H_{F}+H_{L}) without the feed member (3).

19. Device according to any one of the preceding claims, **characterised in that** the feed device (3-7) includes a manually operable actuating element (6), actuation of which effects the feed movement, and **in that** the actuating element (6) is fixed by means of a releasable latching connection (25, 26) to either the dosing device (8) or the casing (1) against the direction of the feed movement when the feed device (3-7) adopts the unblocking position.

20. Device according to any one of the preceding claims, **characterised in that** the locking engagement is automatically released when the feed device (3-7) is moved to the unblocking position.

21. Device according to any one of the preceding claims, **characterised in that** the feed device (3-7) moves to the unblocking position through the feed movement.

22. Device according to any one of the preceding claims, **characterised in that** the dosing device (8) can execute the dosing movement in a freely selectable manner in the direction of an increase and in the direction of a reduction of the product dose.

## Revendications

1. Dispositif pour administrer un produit injectable, comprenant :
a) un boîtier (1) comprenant un réservoir (2) pour le produit,
b) un dispositif de transport (3-7) qui peut être déplacé d'une position de relâchement à une position de déclenchement et qui effectue, à partir de la position de déclenchement, un mouvement de translation par le biais duquel une dose de produit définie est déversée du réservoir (2),
c) et un dispositif de dosage (8) qui peut effectuer un mouvement de dosage pour déterminer la dose de produit dans des positions de dosage respectives prédéterminées par une encoche d'arrêt, lorsque le dispositif de transport (3-7) est en position de relâchement, **caractérisé en ce que**
d) le dispositif de transport (3-7) en position de déclenchement et lors du déplacement en position de déclenchement est couplé au dispositif de dosage (8) par le biais d'un engrènement de blocage qui bloque le dispositif de dosage (8) dans la position de dosage respective définie contre des mouvements de dosage.

2. Dispositif selon la revendication précédente, **caractérisé en ce que** le dispositif de dosage (8) forme une butée de dosage (27) réglable par le mouvement de dosage qui limite le mouvement du dispositif de transport (3-7) de la position de relâchement à la position de déclenchement et détermine ainsi la longueur de course (H_{F}) du mouvement de translation.

3. Dispositif selon la revendication précédente, **caractérisé en ce que** plusieurs butées individuelles forment conjointement la butée de dosage réglable, **en ce que**, pour chaque position se dosage, seule une butée ou un groupe de butées limite le mouvement du dispositif de transport et **en ce que**, pour les doses de produit réglables qui correspondent aux positions de dosage, au moins l'une des butées est prévue, respectivement.

4. Dispositif selon la revendication 2, **caractérisé en ce que** la butée de dosage (27) s'étend en continu avec une inclinaison en direction du mouvement de translation du dispositif de transport (3-7).

5. Dispositif selon l'une quelconque des revendications précédentes, **caractérisé en ce que**, dans les positions de dosage, respectivement un élément choisi parmi le dispositif de transport (3-7) et le dispositif de dosage (8) forme une glissière (9) et l'autre forme un élément d'engrènement (7a ; 7b) et que l'élément d'engrènement (7a ; 7b) est conduit dans l'engrènement de blocage le long de la glissière (9) en direction du déplacement du dispositif de transport (3-7) qui s'effectue en position de déclenchement.

6. Dispositif selon la revendication précédente, **caractérisé en ce que** la glissière (9) et l'élément d'engrènement (7a ; 7b) ne sont pas asservis l'un à l'autre en position de relâchement du dispositif de transport (3-7).

7. Dispositif selon la revendication 5, **caractérisé en ce que** la glissière (9) et l'élément d'engrènement (7a) sont, en position de relâchement du dispositif de transport (3-7), dans une encoche d'arrêt déblocable.

8. Dispositif selon la revendication précédente, **caractérisé en ce que** l'encoche d'arrêt de la glissière (9) et de l'élément d'engrènement (7a) se renforce lors du déplacement qui a lieu en position de déclenchement du dispositif de transport (3-7) en engrènement de blocage.

9. Dispositif selon l'une quelconque des quatre revendications précédentes, **caractérisé en ce que** le mouvement de dosage comprend un mouvement rotatif du dispositif de dosage (8) par rapport au dispositif de transport (3-7) autour d'un axe de rotation (R), le mouvement de translation comprend un mouvement du dispositif de transport (3-7) par rapport au dispositif de dosage (8) le long de l'axe de rotation (R), un élément choisi parmi le dispositif de transport (3-7) et le dispositif de dosage (8) entoure l'autre au moins en partie, et **en ce que** la glissière (9) et l'élément d'engrènement (7a ; 7b) sont prévus sur des surfaces de revêtement opposées orientées l'une en face de l'autre du dispositif de transport (3-7) et du dispositif de dosage (8).

10. Dispositif selon la revendication précédente, **caractérisé en ce qu'**au moins un l'élément choisi parmi la glissière (9) et l'élément d'engrènement (7a) est disposé radialement de façon mobile par rapport à la surface de revêtement sur lequel elle ou il est prévu(e) et est déplacé par le biais du mouvement, qui s'effectue en position de déclenchement, du dispositif de transport (3-7) sur la surface de revêtement opposée et ainsi dans l'engrènement de blocage.

11. Dispositif selon l'une quelconque des revendications 2 à 4 avec l'une des revendications 5 à 10, **caractérisé en ce que** le dispositif de dosage (8) choisi parmi l'élément d'engrènement (7a ; 7b) et la glissière (9) forment en outre la butée de dosage (27).

12. Dispositif selon l'une quelconque des sept revendications précédentes, **caractérisé en ce qu'**un élément choisi parmi l'élément d'engrènement (7a) et la glissière (9) est relié au moyen d'une articulation (14, 21) de façon mobile avec le dispositif de transport (3-7).

13. Dispositif selon l'une quelconque des huit revendications précédentes, **caractérisé en ce que** le dispositif de transport (3-7) comprend un élément d'actionnement (6) actionnable manuellement dont l'action entraîne le mouvement de translation et **en ce que** l'élément d'actionnement (6) et un élément choisi parmi un élément d'engrènement (7b) et la glissière (9) sont formés conjointement en une pièce.

14. Dispositif selon l'une quelconque des revendications 5 à 12, **caractérisé en ce que** le dispositif de transport (3-7) comprend un élément d'actionnement (6) actionnable manuellement dont l'action entraîne le mouvement de translation et **en ce que** l'élément d'actionnement (6) est relié à un élément choisi parmi un élément d'engrènement (7a) et une glissière (9) par le biais d'une articulation (17, 20) qui convertit un mouvement, effectué par l'actionnement, de l'élément d'actionnement (6) en un mouvement d'un élément choisi parmi un élément d'engrènement (7a) et la glissière (9) qui est relié par le biais de l'articulation (17, 20) à l'élément d'actionnement (6).

15. Dispositif selon la revendication précédente, **caractérisé en ce que** l'articulation (17, 20) est une articulation cylindrique, et un élément d'articulation (17) de l'élément d'actionnement (6) et un élément d'articulation (20) de l'élément choisi parmi l'élément d'engrènement (7a) et la glissière (9) peuvent être déplacés l'un par rapport à l'autre dans l'articulation (17, 20) de préférence dans la sens du mouvement de translation du dispositif de transport (3-7) et dans le sens contraire de celui-ci.

16. Dispositif selon l'une des deux revendications précédentes, **caractérisé en ce que** l'élément d'actionnement (6) entraîne, par le biais d'un élément choisi parmi l'élément d'engrènement (7a) et la glissière (9) qui est relié à l'élément d'actionnement (6) par le biais de l'articulation (17, 20), le mouvement de translation du dispositif de transport (3-7) et/ou son mouvement en position de déclenchement.

17. Dispositif selon l'une quelconque des revendications précédentes, **caractérisé en ce que** le dispositif de transport (3-7) comprend un élément d'actionnement actionnable manuellement dont l'action entraîne le mouvement de translation et **en ce que** la longueur de course (H_{F} + H_{L}) d'un mouvement pouvant être effectué par l'action de l'élément d'actionnement (6) est supérieur à la longueur de course (H_{F}) du mouvement de translation correspondant au déversement complet de la dose de produit définie.

18. Dispositif selon la revendication précédente, **caractérisé en ce que** le dispositif de transport (3-7) présente au moins un élément de transport (3) disposé dans le réservoir (2) qui effectue le mouvement de translation et **en ce que** l'élément d'actionnement (6) est couplé à l'élément de transport (3) de telle sorte que, sur une partie (H_{F}) de la longueur de course (H_{F} + H_{L}) de son mouvement, il emporte l'élément de transport (3) et qu'il parcourt l'autre partie (H_{L}) de la longueur de course de son mouvement (H_{F} + H_{L}) sans l'élément de transport (3).

19. Dispositif selon l'une quelconque des revendications précédentes, **caractérisé en ce que** le dispositif de transport (3-7) comprend un élément d'actionnement (6) actionnable manuellement dont l'action entraîne le mouvement de translation et **en ce que** l'élément d'actionnement (6) est fixé au moyen d'une liaison d'arrêt déblocable (25, 26) à un élément choisi parmi le dispositif de dosage (8) et le boîtier (1) dans le sens contraire du mouvement de translation, lorsque le dispositif de transport (3-7) adopte la position de relâchement.

20. Dispositif selon l'une quelconque des revendications précédentes, **caractérisé en ce que** l'engrènement de blocage se débloque automatiquement lorsque le dispositif de transport (3-7) est déplacé en position de relâchement.

21. Dispositif selon l'une quelconque des revendications précédentes, **caractérisé en ce que** le dispositif de transport (3-7) se déplace par le mouvement de translation, en position de relâchement.

22. Dispositif selon l'une quelconque des revendications précédentes, **caractérisé en ce que** le dispositif de dosage (8) peut effectuer facultativement le mouvement de dosage dans le sens d'un accroissement et dans le sens d'une diminution de la dose de produit.
